# FASCICULE DE BREVET EUROPEEN

(11) **EP 2 008 599 B1**
(45) Date de publication et mention de la délivrance du brevet: **26.08.2009**
(21) Numéro de dépôt: 08290562.1
(22) Date de dépôt: 16.06.2008
(51) Int. Cl.: A61B 17/70, A61B 17/80

(54) **Système d'ostéosynthèse par plaque et vis osseuses**
Osteosynthesesystem mit Hilfe von Knochenplatten und -schrauben
Osteosynthesis system using bone plates and screws

(30) Priorité: 25.06.2007 FR 0704530
(43) Date de publication de la demande: 31.12.2008
(73) Titulaire: Razian, Hassan, 94240 l'Hay les Roses (FR)
(72) Inventeur: Razian, Hassan, 94240 l'Hay les Roses (FR)
(74) Mandataire: Flavenot, Bernard

(56) Documents cités:
- US-A- 5 810 823
- US-A- 5 879 389
- US-A- 6 102 951
- US-A1- 2003 083 660

## Description

La présente invention concerne les systèmes d'ostéosynthèse comportant une plaque et au moins une vis osseuse, qui trouvent des applications particulièrement avantageuses comme systèmes d'ostéosynthèse entre au moins deux os, ou portions d'os, tels que des vertèbres.

Il est connu des systèmes de fixation sur un corps de toute nature par vissage d'une vis avec l'interposition, entre la tête de la vis et le corps dans lequel doit être vissée la tige filetée de la vis, une rondelle ou tout autre élément comme une plaque de liaison ou analogue.

Ces systèmes de fixation posent essentiellement deux types de problèmes. L'un de ces problèmes est celui du maintien en place à la fois de la vis et de la plaque ou rondelle lors du vissage de la vis. L'autre est celui de la fiabilité du vissage dans le temps, c'est-à-dire le risque du dévissage de la vis pour quelque raison que ce soit, par exemple sous l'action de vibrations ou analogues, et même le risque que la vis ne se détache totalement du corps et se perde.

Ce second problème est particulièrement grave dans le cas de la mise en place d'une plaque pour ostéosynthèse, par exemple rachidienne, car le dévissage de la vis ou des vis peut être très dangereux pour le patient dans le corps duquel ces vis ont été implantées pour maintenir la plaque.

Il existe déjà de nombreux dispositifs qui permettent de réaliser des fixations par vissage au moyen de vis de type imperdable. Cependant, tous ces dispositifs sont relativement complexes et donc onéreux.

Aussi, la présente invention a-t-elle pour but de réaliser un système d'ostéosynthèse comportant une plaque et au moins une vis osseuse qui pallie au moins en grande partie les inconvénients mentionnés ci-dessus des systèmes similaires de l'art antérieur.

Un système d'ostéosynthèse selon le préambule de la revendication 1 est décrit dans US-A-5 810 823.

Plus précisément, la présente invention a pour objet un système d'ostéosynthèse comportant une plaque et au moins une vis osseuse tel que défini dans la revendication 1 annexée.

D'autres caractéristiques et avantages de l'invention apparaîtront au cours de la description suivante donnée en regard des dessins annexés à titre illustratif mais nullement limitatif, dans lesquels :

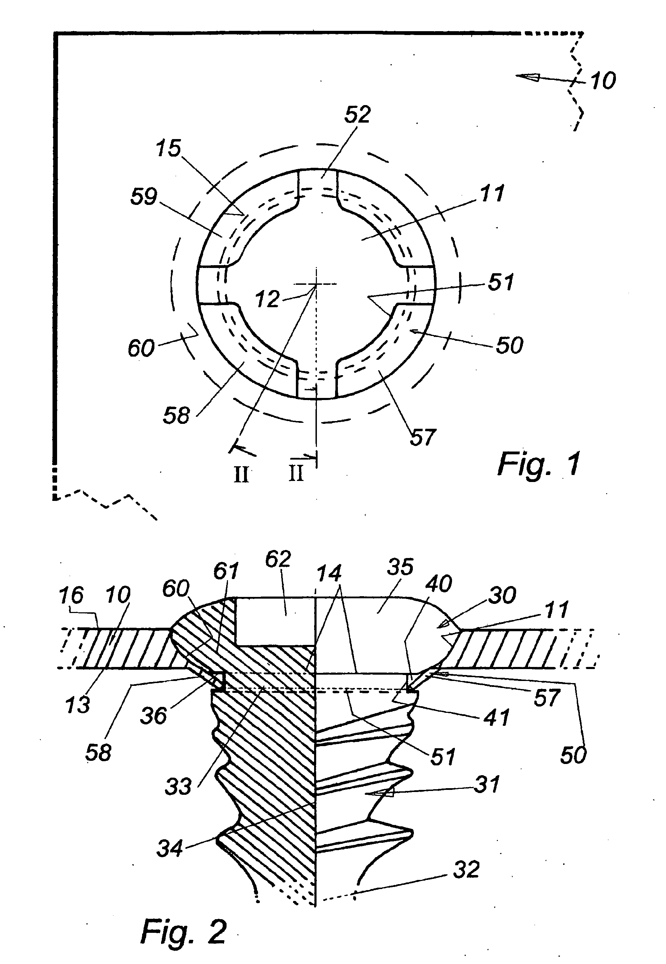

Les figures 1 et 2 représentent deux vues d'un mode de réalisation du système d'ostéosynthèse selon l'invention comportant une plaque et vis osseuse, la figure 1 étant une vue de dessus d'une partie de la plaque et la figure 2 une vue en coupe et partiellement en écorché d'une plaque et d'une vis osseuse assemblées l'une avec l'autre en accord avec la représentation selon la figure 1, cette coupe et cet écorché étant référencés II-II sur la figure 1.

En référence aux deux figures prises ensemble ou séparément, la présente invention concerne un système d'ostéosynthèse comportant une plaque 10 et au moins une vis osseuse 30.

La vis osseuse 30 comprend une tige 31 à filetage osseux, auto-taraudant ou non, du type "filetage cortical ou spongieux", ou une combinaison des deux, cette tige étant par exemple en titane ou analogue, définie selon un axe longitudinal 34, et comportant une extrémité distale pénétrante 32 et une extrémité opposée proximale 33. La vis osseuse comprend également une tête d'épaulement 35 solidaire de l'extrémité proximale 33 de la tige 31.

Le filetage de la tige peut être avantageusement, comme illustré sur la figure 2, à fond de filet sur une surface conique dont le sommet est tourné vers l'extrémité pénétrante 32 de la tige, alors que les sommets des filets sont sur une surface cylindrique de révolution.

Quant à la plaque 10, elle comporte au moins une percée traversante 11 apte à recevoir la vis osseuse. Cette percée traversante est définie selon une direction axiale 12 sensiblement perpendiculaire au plan général de la plaque 10 et débouche sur une première 13 des deux faces de la plaque par une ouverture distale 14. Elle a une section hors-tout supérieure à la section transversale de la tige 31 définie perpendiculairement à l'axe longitudinal 34 prise au niveau de son extrémité proximale 33 et inférieure à la section hors-tout de la tête d'épaulement 35 définie dans un plan perpendiculaire à ce même axe longitudinal 34.

Selon une caractéristique importante de l'invention, le système comporte en outre une gorge annulaire 40 réalisée sur l'extrémité proximale 33 de la tige 31 en étant sensiblement centrée sur l'axe longitudinal 34, et une membrane 50 solidaire fixement de la plaque 10 en étant située dans la percée traversante 11.

Cette membrane peut être réalisée d'une seule pièce avec la plaque 10 ou rapportée, par exemple par emboîtement en force, vissée ou analogue dans la paroi latérale de la percée traversante 11.

Cette membrane 50 présente une épaisseur au plus égale, et très avantageusement inférieure (figure 2), à la hauteur de la gorge 40 définie selon l'axe longitudinal 34. Elle comporte un orifice 51 (figure 1) réalisé sensiblement centré sur la direction axiale 12 de la percée traversante 11 et ayant une section inférieure à la section du fond 41 de la gorge 40.

La membrane 50 comporte en outre au moins une découpe 52, figure 1, sensiblement radiale débouchant au moins dans son orifice central 51 et, sans que cela soit obligatoire bien qu'avantageux, cette découpe 52 commence sensiblement sur le bord 15 de la percée traversante 11.

En fait, comme illustré sur les figures, la membrane 50 comporte avantageusement une pluralité de découpes 52 sensiblement radiales débouchant au moins dans son orifice central 51 et régulièrement réparties. Dans le mode de réalisation illustré sur la figure 1, la membrane comporte quatre découpes 52 qui la partagent en quatre ailettes 57, 58, 59, ...

Selon une réalisation préférentielle sur le plan de la fabrication du système, plus particulièrement des vis osseuses 30, l'un des deux bords latéraux de la gorge annulaire 40 est défini par la paroi 36 de la tête d'épaulement 35 qui est solidaire de l'extrémité proximale 33 de la tige 31.

En outre, de façon avantageuse et préférentielle, la membrane 50 est solidaire de la plaque 10 en étant située sensiblement dans le plan de l'ouverture distale 14.

Il est aussi possible et avantageux, pour faciliter la fixation du système sur un os ou une portion d'os qui n'a pas une surface très plane, comme une vertèbre ou analogue qui est recouverte d'éléments comme des processus épineux ou analogues, que la percée traversante 11 comporte en outre un alésage 60 de forme concave sensiblement hémisphérique ou analogue, débouchant sur la face 16 de la plaque 10 opposée à la première face 13, la tête d'épaulement 35 comportant alors, figure 2, une partie convexe 61 sensiblement complémentaire de cet alésage concave 60 pour permettre à cette tête d'épaulement 35 de pivoter dans l'alésage par rapport à la plaque 10 et donc à la vis osseuse de pouvoir être plus facilement vissée dans l'os selon une orientation préférentielle.

Aussi, de façon préférentielle, bien que non spécifiquement illustré sur la figure 2, l'épaisseur de la plaque 10 est au moins égale à la hauteur de la tête d'épaulement 35, de façon que cette tête d'épaulement soit entièrement située dans l'épaisseur de la plaque pour, de façon connue, éviter de blesser les chairs du patient.

Comme de façon connue également, sont aussi prévus des moyens 62 pour permettre de commander la rotation de la vis osseuse 30 autour de l'axe longitudinal 34, par exemple, comme illustré sur la figure 2, un orifice cylindrique à section polygonale du type "six pans" ou analogue apte à recevoir un tournevis ou analogue.

Le système selon l'invention dont un mode de réalisation a été décrit ci-dessus en regard des figures 1 et 2, s'utilise de la façon suivante :
Lorsqu'un praticien veut solidariser deux portions d'os l'une avec l'autre, il choisit par exemple une plaque 10 comportant quatre percées traversantes 11 et quatre vis osseuses 30 correspondantes pour coopérer avec ces quatre percées.

En général, il visse deux vis osseuses à travers deux percées de la plaque, dans une portion d'os, puis les deux autres vis osseuses à travers les deux autres percées, dans l'autre portion d'os.

Cette technique est connue et ne sera pas plus amplement développée ici.

Il est cependant rappelé que le problème principal qui peut se poser dans un tel système d'ostéosynthèse est le fait que les vis peuvent se dévisser leur dévissage engendrant un déplacement des deux portions d'os l'une par rapport à l'autre, et peuvent même migrer dans le corps du patient en créant des douleurs pour le patient sur lequel le système d'ostéosynthèse a été implanté.

Avec le système d'ostéosynthèse selon l'invention, cet inconvénient est évité.

En effet, lorsque le praticien commence à visser une vis osseuse 30, il l'introduit dans une percée traversante 11 par son extrémité distale 32 sensiblement suivant la direction axiale, la plaque 10 étant au contact de la portion osseuse. Au fur et à mesure que le praticien procède au vissage de la vis osseuse 30, cette dernière pénètre dans l'os et sa tête d'épaulement 35 se rapproche de la plaque 10. Quand la zone de la tige osseuse 31 qui a la même section transversale que l'orifice 51 de la membrane 50 arrive au niveau de cette membrane et que la tige continue à être vissée, les ailettes 57, 58, 59, ... se rabattent en direction de l'extrémité distale 52 de la tige.

A la fin de son vissage, la gorge annulaire 40 arrive au niveau des extrémités libres de ces ailettes qui tombent dans le fond de la gorge en se redressant partiellement mais en conservant une inclinaison vers l'extrémité distale 52 de la tige 31. Cette inclinaison est due au fait que la section du fond 41 de la gorge est supérieure à la section de l'orifice 51 avant la déformation de la membrane comme décrit ci-dessus.

L'inclinaison des ailettes telle que définie ci-dessus empêche le dévissage accidentel d'une telle vis osseuse.

Néanmoins, il est possible de procéder volontairement au dévissage de cette vis, simplement en exerçant une force de dévissage assez intense pour, dans un premier temps déformer élastiquement les ailettes et, dans un second temps, leur faire subir une inclinaison inverse à celle décrite ci-avant, c'est-à-dire vers la tête d'épaulement 35, pour qu'elles se dégagent de la gorge annulaire 40 et laissent passer la tige filetée jusqu'à son dévissage complet. Il est possible d'obtenir le même résultat en dévissant toutes les vis, les vis et la plaque restant attachées les unes aux autres et la membrane logée dans la gorge annulaire 40.

## Revendications

1. Système d'ostéosynthèse comportant une plaque (10) et au moins une vis osseuse (30), dans lequel :
• ladite vis osseuse (30) comprend une tige (31) à filetage osseux définie selon un axe longitudinal (34), ladite tige (31) comportant une extrémité distale pénétrante (32) et une extrémité opposée proximale (33), et une tête d'épaulement (35) solidaire de l'extrémité proximale (33) de la tige (31), et
• ladite plaque (10) comporte au moins une percée traversante (11) apte à recevoir ladite vis osseuse et définie selon une direction axiale (12) sensiblement perpendiculaire au plan général de ladite plaque (10), ladite percée traversante (11) débouchant sur une première (13) des deux faces de ladite plaque par une ouverture distale (14) et ayant une section hors-tout supérieure à la section transversale de ladite tige (31) définie perpendiculairement au dit axe longitudinal (34) au niveau de son extrémité proximale (33) et inférieure à la section hors-tout de ladite tête d'épaulement (35) définie dans un plan perpendiculaire au même axe longitudinal (34),
**caractérisé par le fait qu'**il comporte en outre :
• une gorge annulaire (40) réalisée sur l'extrémité proximale (33) de ladite tige (31) en étant sensiblement centrée sur l'axe longitudinal (34), et
• une membrane (50) solidaire de la plaque (10) en étant située dans ladite percée traversante (11), ladite membrane ayant une épaisseur au plus égale à la hauteur de ladite gorge (40) définie selon l'axe longitudinal (34) et comportant un orifice (51) réalisé sensiblement centré sur la direction axiale (12) de la percée (11) et ayant une section inférieure à la section du fond (41) de ladite gorge (40), ladite membrane (50) comportant en outre au moins une découpe (52) sensiblement radiale débouchant au moins dans son orifice central (51).

2. Système selon la revendication 1, **caractérisé par le fait que** ladite découpe (52) commence sensiblement sur le bord (15) de ladite percée traversante (11).

3. Système selon l'une des revendications 1 et 2, **caractérisé par le fait que** ladite membrane (50) comporte une pluralité de découpes (52) sensiblement radiales débouchant au moins dans son orifice central (51) et régulièrement réparties.

4. Système selon l'une des revendications 1 à 3, **caractérisé par le fait que** l'épaisseur de ladite membrane (50) est inférieure à la hauteur de ladite gorge annulaire (40).

5. Système selon l'une des revendications précédentes, **caractérisé par le fait qu'**un bord latéral de ladite gorge annulaire (40) est défini par la paroi (36) de la tête d'épaulement (35) solidaire de l'extrémité proximale (33) de la tige (31).

6. Système selon l'une des revendications précédentes, **caractérisé par le fait que** ladite membrane (50) est solidaire de la plaque (10) en étant située sensiblement dans le plan de l'ouverture distale (14).

7. Système selon la revendication 6, **caractérisé par le fait que** ladite percée traversante (11) comporte un alésage (60) de forme concave débouchant sur la face (16) de la plaque (10) opposée à la première face (13), la tête d'épaulement (35) comportant une partie convexe (61) sensiblement complémentaire de cet alésage concave (60) pour permettre à cette tête d'épaulement (35) de pivoter dans l'alésage par rapport à ladite plaque (10).

8. Système selon l'une des revendications précédentes, **caractérisé par le fait qu'**il comporte des moyens (62) pour commander la rotation de ladite vis osseuse (30) autour de l'axe longitudinal (34).

9. Système selon l'une des revendications précédentes, **caractérisé par le fait que** l'épaisseur de ladite plaque (10) est au moins égale à la hauteur de la tête d'épaulement (35).

## Claims

1. An osteosynthesis system comprising a plate (10) and at least one bone screw (30), wherein:
said bone screw (30) comprises a bone-threaded shank (31) defining a longitudinal axis (34), said shank (31) having a penetrating distal end (32) and an opposite proximal end (33), and a shouldered head (35) secured to the proximal end (33) of the shank (31); and
said plate (10) includes at least one through hole (11) suitable for receiving said bone screw and defined along an axial direction (12) that is substantially perpendicular to the general plane of said plate (10), said through hole (11) opening out into a first face (13) of the two faces of said plate via a distal opening (14) and having an overall section that is greater than the cross-section of said shank (31) defined perpendicularly to said longitudinal axis (34) at its proximal end (33), and less than the overall section of said shouldered head (35) defined in a plane perpendicular to the same longitudinal axis (34);
the system being **characterized by** the fact that it further comprises:
■ an annular groove (40) formed in the proximal end (33) of said shank (31), being substantially centered on said longitudinal axis (34); and
■ a membrane (50) integral with the plate (10) being situated in said through hole (11), said membrane presenting thickness no greater than the height of said groove (40) defined along the longitudinal axis (34) and including an orifice (51) substantially centered on the axial direction (12) of the hole (11) and having a section that is less than the section of the bottom (41) of said groove (40), said membrane (50) further including at least one substantially radial cut (52) opening out at least into its central orifice (51).

2. A system according to claim 1, **characterized by** the fact that said cut (52) begins substantially at the edge (15) of said through hole (11).

3. A system according to claim 1 or claim 2, **characterized by** the fact that said membrane (50) includes a plurality of regularly-distributed substantially-radial cuts (52) opening out at least into its central orifice (51).

4. A system according to any one of claims 1 to 3, **characterized by** the fact that the thickness of said membrane (50) is less than the height of said annular groove (40).

5. A system according to any preceding claim, **characterized by** the fact that one lateral side of said annular groove (40) is defined by the wall (36) of the shouldered head (35) secured to the proximal end (33) of the shank (31).

6. A system according to any preceding claim, **characterized by** the fact that said membrane (50) is integral with the plate (10), being situated substantially in the plane of the distal opening (14).

7. A system according to claim 6, **characterized by** the fact that said through hole (11) includes a bore (60) of concave shape opening into the face (16) of the plate (10) that is opposite from the first face (13), the shouldered head (35) including a convex portion (61) substantially complementary to said concave bore (60) so as to enable said shouldered head (35) to pivot in the bore relative to said plate (10).

8. A system according to any preceding claim, **characterized by** the fact that it includes means (62) for controlling rotation of said bone screw (30) about the longitudinal axis (34).

9. A system according to any preceding claim, **characterized by** the fact that the thickness of said plate (10) is equal to not less than the height of the shouldered head (35).

## Patentansprüche

1. Osteosynthese-System, das eine Platte (10) und wenigstens eine Knochenschraube (30) umfasst, wobei:
- die Knochenschraube (30) einen Stift (31) mit Knochengewinde aufweist, der längs einer longitudinalen Achse (34) definiert ist, wobei der Stift (31) ein eindringendes distales Ende (32) und ein gegenüberliegendes proximales Ende (33) sowie einen mit dem proximalen Ende (33) des Stifts (31) fest verbundenen Anschlagkopf (35) umfasst, und
- die Platte (10) wenigstens ein Durchgangsloch (11) aufweist, das die Knochenschraube aufnehmen kann und längs einer axialen Richtung (12), die zu der allgemeinen Ebene der Platte (10) im Wesentlichen senkrecht ist, definiert ist, wobei das Durchgangsloch (11) in eine erste (13) der zwei Flächen der Platte durch eine distale Öffnung (14) mündet und einen Querschnitt über alles besitzt, der größer als der transversale Schnitt des Stifts (31) ist, der auf Höhe seines proximalen Endes (33) senkrecht zu der longitudinalen Achse (34) definiert ist, und kleiner als der Querschnitt über alles des Anschlagkopfes (35) ist, der in einer Ebene senkrecht zu derselben longitudinalen Achse (34) definiert ist,
**dadurch gekennzeichnet, dass es** außerdem umfasst:
- eine Ringnut (40), die am proximalen Ende (33) des Stifts (31) verwirklicht ist und auf die longitudinale Achse (34) im Wesentlichen zentriert ist, und
- eine Membran (50), die mit der Platte (10) fest verbunden ist und sich in dem Durchgangsloch (11) befindet, wobei die Membran eine Dicke besitzt, die höchstens gleich der Höhe der Nut (40) ist, die längs der longitudinalen Achse (34) definiert ist, und eine Öffnung (51) aufweist, die im Wesentlichen zentriert auf die axiale Richtung (12) des Lochs (11) verwirklicht ist und einen Querschnitt hat, der kleiner als der Querschnitt des Bodens (41) der Nut (40) ist, wobei die Membran (50) außerdem wenigstens einen im Wesentlichen radialen Ausschnitt (52) aufweist, der zumindest in ihre mittige Öffnung (51) mündet.

2. System nach Anspruch 1, **dadurch gekennzeichnet, dass** der Ausschnitt (52) im Wesentlichen am Rand (15) des Durchgangslochs (11) beginnt.

3. System nach einem der Ansprüche 1 und 2, **dadurch gekennzeichnet, dass** die Membran (50) mehrere im Wesentlichen radiale Ausschnitte (52) aufweist, die zumindest in ihre mittige Öffnung (51) münden und regelmäßig verteilt sind.

4. System nach einem der Ansprüche 1 bis 3, **dadurch gekennzeichnet, dass** die Dicke der Membran (50) kleiner als die Höhe der Ringnut (40) ist.

5. System nach einem der vorhergehenden Ansprüche, **dadurch gekennzeichnet, dass** ein seitlicher Rand der Ringnut (40) durch die Wand (36) des Anschlagkopfes (35), der mit dem proximalen Ende (33) des Stifts (31) fest verbunden ist, definiert ist.

6. System nach einem der vorhergehenden Ansprüche, **dadurch gekennzeichnet, dass** die Membran (50) mit der Platte (10) fest verbunden ist und sich im Wesentlichen in der Ebene der distalen Öffnung (14) befindet.

7. System nach Anspruch 6, **dadurch gekennzeichnet, dass** das Durchgangsloch (11) eine Bohrung (60) mit konkaver Form aufweist, die in die Fläche (16) der Platte (10) gegenüber der ersten Fläche (13) mündet, wobei der Anschlagkopf (35) einen konvexen Teil (61) aufweist, der zu dieser konkaven Bohrung (60) im Wesentlichen komplementär ist, um diesem Anschlagkopf (35) zu ermöglichen, in der Bohrung in Bezug auf die Platte (10) zu schwenken.

8. System nach einem der vorhergehenden Ansprüche, **dadurch gekennzeichnet, dass** es Mittel (62) umfasst, um die Drehung der Knochenschraube (30) um die longitudinale Achse (34) zu steuern.

9. System nach einem der vorhergehenden Ansprüche, **dadurch gekennzeichnet, dass** die Dicke der Platte (10) wenigstens gleich der Höhe des Anschlagkopfes (35) ist.
